# EUROPEAN PATENT SPECIFICATION

(11) **EP 3 694 522 B1**
(45) Date of publication and mention of the grant of the patent: **26.11.2025**
(21) Application number: 18866268.8
(22) Date of filing: 08.10.2018
(51) Int. Cl.: A61K 9/20, A61K 31/513, A61K 31/5365, A61P 31/18, A61K 9/24

(54) **BI-LAYER PHARMACEUTICAL TABLET FORMULATION**
ZWEISCHICHTIGE PHARMAZEUTISCHE TABLETTENFORMULIERUNG
FORMULATION DE COMPRIMÉ PHARMACEUTIQUE BICOUCHE

(30) Priority: 13.10.2017 US 201762571863 P; 23.02.2018 US 201862634317 P
(43) Date of publication of application: 19.08.2020
(73) Proprietor: VIIV Healthcare Company, Wilmington, Delaware 19808 (US)
(72) Inventor: KAYE, Jonathan, Louis, Ware Hertfordshire SG12 0DP (GB)
(74) Representative: Wilkinson, Johanna Elise
(86) International application number: PCT/US2018/054825
(87) International publication number: WO 2019/074826

(56) References cited:
- WO-A1-2014/064409
- WO-A1-2014/064409
- US-A1- 2017 189 402
- US-A1- 2017 217 987
- CAHN PEDRO ET AL: "Dolutegravir-lamivudine as initial therapy in HIV-1 infected, ARV-naive patients, 48-week results of the PADDLE (Pilot Antiretroviral Design with Dolutegravir LamivudinE) study", vol. 20, no. 1, 1 January 2017 (2017-01-01), London, UK, pages 21678, XP055784450, ISSN: 1758-2652, Retrieved from the Internet <URL:https://api.wiley.com/onlinelibrary/tdm/v1/articles/10.7448%2FIAS.20.01.21678> DOI: 10.7448/IAS.20.01.21678
- ANONYMOUS: "Highlights of prescribing information for EPIVIR", 1 September 2017 (2017-09-01), XP055816986, Retrieved from the Internet <URL:https://www.accessdata.fda.gov/drugsatfda_docs/label/2017/020564s37_020596s036lbl.pdf> [retrieved on 20210623]
- ANONYMOUS: "Highlights of prescribing information for TIVICAY", 1 March 2017 (2017-03-01), XP055816989, Retrieved from the Internet <URL:https://www.accessdata.fda.gov/drugsatfda_docs/label/2017/204790s013lbl.pdf> [retrieved on 20210623]
- ANONYMOUS: "Dolutegravir (Tivacy) for HIV", MEDICAL LETTER ON DRUGS AND THERAPEUTICS, vol. 55, no. 1426, 30 September 2013 (2013-09-30), pages 77 - 80, XP009519910, Retrieved from the Internet <URL:https://secure.medicalletter.org/article-share?a=1426b&p=tml&title=Dolutegravir%20(Tivicay)%20for%20HIV&cannotaccesstitle=1>

## Description

### FIELD OF THE INVENTION

The present invention relates to a novel bi-layer pharmaceutical tablet formulation for use in treating HIV. In particular, the invention relates to a two-drug tablet formulation comprising the integrase strand transfer inhibitor dolutegravir sodium (abbreviated "DTG Na") with the nucleoside reverse transcriptase translocation inhibitor (NRTTI) lamivudine (also known as "3TC") and methods of using such a formulation in the treatment of conditions in which the inhibition of the HIV integrase or reverse transcriptase is beneficial, e.g., HIV.

### BACKGROUND OF THE INVENTION

Over the past decades, advances in highly-active antiretroviral therapies (ARTs) have improved treatment efficacy for patients with HIV, improving patient survival and quality of life. However, proper adherence to treatment regimens remains a challenge where poor compliance can result in treatment failure and the emergence of drug-resistant mutations. To help aid adherence, simplified treatments are under investigation. Both oral and long-acting injectable ART may provide patients with a convenient and discreet approach to manage HIV infection.

Dolutegravir is an integrase strand transfer inhibitor (INSTI) that exhibits subnanomolar potency and antiviral activity against a broad range of HIV-1 strains. Oral administration of dolutegravir has exhibited acceptable safety and tolerability profiles and few drug-drug interactions. To minimize the emergence of drug resistant mutations, dolutegravir is currently administered in combination with one or more additional anti-HIV agents, most typically in the three-way combination of dolutegravir, abacavir, and lamivudine, known as TRIUMEQ.

Dolutegravir, in two-way combination with lamivudine, is currently under non-inferiority clinical studies against three-way treatments to evaluate long-term antiviral activity, tolerability, and safety parameters. The two-way combination (two-drug regimen) has potential to reduce drug burden, potential toxicities, and potential interactions in comparison to current three-way regimens.

WO2014/064409 discloses pharmaceutical compositions comprising anti-retroviral agents, the manufacturing process thereof and use of the said composition for the prevention, treatment or prophylaxis of retroviral disease in the patients in need thereof.

It is desired to provide a pharmaceutical composition that provides a single dosage form that includes both dolutegravir and lamivudine in a safe and efficacious two-drug regimen.

### SUMMARY OF THE INVENTION

The invention is set out in the appended set of claims. Described herein is a bi-layer tablet formulation of lamivudine and dolutegravir sodium having favorable dissolution characteristics and resulting in favorable phamacokinetic properties previously only available in distinct dosage forms.

The tablet as described herein is a bi-layer tablet that comprises a first layer comprising lamivudine, and a second layer comprising dolutegravir sodium. According to an embodiment, the first layer comprises about 300 mg lamivudine, and at least one additional excipient, and the second layer comprises about 50 mg dolutegravir, and at least one additional excipient. According to another embodiment, the first layer comprises about 300 mg lamivudine, a filler, a disintegrant, and a lubricant. According to an embodiment, the first layer comprises about 300 mg lamivudine, about 277.5 mg microcrystalline cellulose, sodium starch glycoate, and magnesium stearate. According to an embodiment, the second layer comprises about 50 mg dolutegravir, one or more diluents, a binder, and a disintegrant. According to an embodiment, the second layer comprises about 50 mg dolutegravir, D-Mannitol, microcrystalline cellulose, povidone, and sodium starch glycoate. According to an embodiment, the tablet further comprising a film coat.

The pharmacokinetics resulting from administration of the tablet to a human are important. Specifically, the tablet advantageously provides pharmacokinetics consistent with previously approved pharmacokinetic profiles such as those for the combined administration of separate dosage forms of dolutegravir sodium and rilpivirine as approved by regulatory agencies, e.g. U.S. F.D.A.. According to an embodiment, the tablet of the invention provides to a patient upon oral administration substantially the same AUC_{(0-∞)} of dolutegravir as the FDA approved 50mg TIVICAY product. According to another embodiment, the tablet of the invention provides to a patient upon oral administration substantially the same AUC_{(0-∞)} of lamivudine as the FDA approved 300mg EPIVIR product. According to another embodiment, the tablet of the invention provides to a fasted patient upon oral administration an AUC_{(0-∞)} between 13.3 and 13.9 mcgh/mL of lamivudine. According to another embodiment, the tablet of the invention provides to a fasted patient upon oral administration an AUC_{(0-∞)} and between 50.5 and 58.9 mcgh/mL of dolutegravir.

The dissolution profile of a combination formulation is important. Here, lamivudine is known to be highly soluble and highly permeable under typical physiologic conditions. By comparison, dolutegravir is only slightly soluble and permeable under typical physiologic conditions. According to an embodiment, the tablet of the invention comprises a first layer comprising about 300 mg lamivudine, and a second layer comprising about 50 mg dolutegravir, wherein about 35% to 40% of the dolutegravir is released in about 60 minutes following contact with a 1.6 pH simulated gastric fluid as measured with USP Apparatus II. According to another embodiment, the dolutegravir release is measured in 500 mL of simulated gastric fluid, at 37.0 +/- 0.5 °C, and paddle speed of 65 rpm.

### DETAILED DESCRIPTION

According to an embodiment of the invention, the bi-layer tablets of the invention are film-coated tablets for oral administration. The tablets contain 52.6 mg dolutegravir sodium which is equivalent to 50 mg dolutegravir free acid and 300 mg lamivudine. The primary components of the uncoated tablet are dolutegravir sodium, lamivudine, D-Mannitol, microcrystalline cellulose, povidone, sodium starch glycolate, sodium stearyl fumarate, and magnesium stearate. An optional film coating may be applied.

According to an embodiment of the invention, there is provided a bi-layer tablet containing the quantity of components shown in Table 1.

**Table 1**

| **Component** | **Exemplary Quantity mg/tablet** | **Exemplary Quantity wt%/wt%** | **Exemplary Range (90%-110%) mg/tablet** | **Exemplary Range (90%-110%) wt/wt%** | **Function** |
|---|---|---|---|---|---|
| Lamivudine | 300.0 | 33.3 | 270.0-330.0 | 30.0-36.7 | Active |
| Dolutegravir Sodium | 52.6 | 5.8 | 47.3-57.8 | 5.3-6.4 | Active |
| Microcrystalline Cellulose | 337.5 | 37.5 | 303.8-371.3 | 33.8-41.3 | Filler |
| Sodium Starch Glycolate | 39.0 | 4.3 | 35.1-42.9 | 3.9-4.8 | Disintegrant |
| Magnesium Stearate | 4.5 | 0.5 | 4.1-4.9 | 0.5-0.5 | Lubricant |
| D-Mannitol | 145.4 | 16.2 | 130.8-159.9 | 14.5-17.8 | Diluent |
| Povidone | 15.0 | 1.7 | 13.5-16.5 | 1.5-1.8 | Binder |
| Sodium Stearyl Fumarate | 6.0 | 0.7 | 5.4-6.6 | 0.6-0.7 | Lubricant |
| Purified Water | q.s. | | q.s. | | Vehicle |

According to another embodiment, there is provided a bi-layer tablet containing the quantity of components shown in Table 1 as a tablet core, further comprising a film coating.

According to another embodiment, there is provided a bi-layer tablet containing the exemplary range wt% of components shown in Table 1. According to a more particular embodiment, there is provided a bi-layer tablet containing the exemplary quantity wt% of components of Table 1.

Dolutegravir inhibits HIV integrase by binding to the integrase active site and blocking the strand transfer step of retroviral deoxyribonucleic acid ("DNA") integration which is essential for the HIV replication cycle. DTG is an integrase strand transfer inhibitor (INSTI). Strand transfer biochemical assays using purified HIV-1 integrase and pre-processed substrate DNA resulted in IC50 (Inhibitory Concentration at 50%) values of 2.7 nM (Kalama and Murphy, Dolutegravir for the Treatment of HIV, 2012 Exp. Op. Invest. Drugs 21(4): 523-530).

The chemical name of dolutegravir is (4R,12aS)-N-[(2,4-difluorophenyl)methyl]-7-hydroxy-4-methyl-6,8-dioxo-3,4,12,12a-tetrahydro-2H-pyrido[5,6]pyrazino[2,6-b][1,3]oxazine-9-carboxamide (CAS Registry Number 1051375-16-6). According to invention, dolutegravir sodium is used. The sodium salt of dolutegravir and a specific crystalline form of this sodium salt are disclosed in U.S. Pat. No. 9,242,986. Unless specified otherwise, the weight (mg) of dolutegravir is based on the weight of dolutegravir in its free form.

Dolutegravir is approved for use in a broad population of HIV-infected patients. Dolutegravir was approved by the FDA in August 2013, by Health Canada in November 2013, and by the EMA in Europe in January 2014 as the pharmaceutical product TIVICAY^{™}, currently available in 50mg, 10mg, and 5mg dosages (measured by weight of dolutegravir free base equivalent). It can be used to treat HIV-infected adults who have never taken HIV therapy (treatment-naive) and HIV-infected adults who have previously taken HIV therapy (treatment-experienced), including those who have been treated with other integrase strand transfer inhibitors.

Dolutegravir sodium is preferably micronized, for instance X₉₀ of 5.7µm-26.3µm, though it has been found that the degree of micronization is not critical to the processability, solubility, dissolution, or bioavailability of dolutegravir sodium according to this invention.

The pharmacokinetic properties of dolutegravir have been evaluated in healthy adult subjects and HIV-1-infected adult subjects. Exposure to dolutegravir was generally similar between healthy subjects and HIV-1-infected subjects. There is a non-linear exposure of dolutegravir following 50 mg twice daily compared with 50 mg once daily in HIV-1-infected subjects (Table 2) attributed to the use of metabolic inducers in the background antiretroviral regimens of subjects receiving dolutegravir 50 mg twice daily in clinical trials.

**Table 2. Dolutegravir Steady-State Pharmacokinetic Parameter Estimates in HIV-1-Infected Adults**

| Parameter | 50 mg Once Daily Geometric Mean^{a} (%CV) | 50 mg Twice Daily Geometric Mean^{b} (%CV) |
|---|---|---|
| AUC(0-24) (mcg.h/mL) | 53.6 (27) | 75.1 (35) |
| Cmax (mcg/mL) | 3.67 (20) | 4.15 (29) |
| Cmin (mcg/mL) | 1.11 (46) | 2.12 (47) |

| | | |
|---|---|---|
| a Based on population pharmacokinetic analyses using data from SPRING-1 and SPRING-2 clinical trials. b Based on population pharmacokinetic analyses using data from VIKING (ING112961) and VIKING-3 clinical trials. | | |

The invented tablet seeks to replicate the relevant AUC, Cmax, and Cmin pharmacokinetic parameters of the approved dolutegravir sodium drug product.

Lamivudine (also known as "3TC"), a synthetic nucleoside analogue with activity against HIV-1 and HBV. The chemical name of lamivudine is (2R,cis)-4-amino-1(2-hydroxymethyl-1,3-oxathiolan-5-yl)-(1H)-pyrimidin-2-one. Lamivudine is the (-) enantiomer of a dideoxy analogue of cytidine. Lamivudine has also been referred to as (-) 2',3'-dideoxy, 3'thiacytidine. It has a molecular formula of C₈H₁₁N₃O₃S. Lamivudine is approved by the FDA under the tradename EPIVIR^{™} (currently marketed in a 300mg dosage form), indicated for use in combination with other antiretroviral agents for the treatment of HIV-1 infection.

The pharmacokinetic information for EPIVIR administered to adults 150 mg twice daily is reported as a geometric mean (95% CI) for AUC(0-12) was 5.53 (4.58, 6.67) mcg.h/mL and for Cmax was 1.40 (1.17, 1.69) mcg/mL. The steady-state pharmacokinetic properties of the EPIVIR 300-mg tablet once daily for 7 days compared with the EPIVIR 150-mg tablet twice daily for 7 days were assessed in a crossover trial in 60 healthy subjects. EPIVIR 300 mg once daily resulted in lamivudine exposures that were similar to EPIVIR 150 mg twice daily with respect to plasma AUC24,ss; however, Cmax,ss was 66% higher and the trough value was 53% lower compared with the 150-mg twice-daily regimen. Thus, as approved, the Cmax and Cmin of lamivudine can be vary within a relatively wide range while still being safe and efficacious.

The invented tablet seeks to replicate the relevant AUC, Cmax, and Cmin pharmacokinetic parameters of the approved lamivudine drug product.

Of note, lamivudine is highly soluble under standard physiological conditions, so dissolution of lamivudine or the lamivudine portion multi-component tablet is not of significant concern.

"Filler" and "Diluent" are used interchangeably herein (and collectively referred to as fillers accept as more particularly described below) to describe materials that increase the bulk of the composition so that the final product has a practical size or volume, for instance for a tablet a practical size for proper compression. Any suitable filler that is compatible with the active ingredient and to good flow properties and dissolution may be utilized. Exemplary fillers include, but are not limited to lactose, sucrose or powdered sugar, mannitol, sorbitol, xylitol, inositol, calcium phosphate, calcium carbonate, calcium sulfate, dry starch, cellulose, including microcrystalline cellulose or silicified microcrystalline cellulose and the like, and combinations thereof.

Preferably microcrystalline cellulose is used as the filler in the lamivudine layer of the invention. The microcrystalline cellulose is preferably present in the lamivudine layer in an amount of 249.5-305.3 mg per uncoated tablet core. Alternatively, the microcrystalline cellulose preferably in the lamivudine layer in the range of 27.7-33.9 wt/wt% of the uncoated tablet core.

Preferably microcrystalline cellulose and D-Mannitol are used as the diluents in the dolutegravir layer. The microcrystalline cellulose is preferably present in the dolutegravir layer in an amount of 47.3-57.9 mg per uncoated tablet core. Alternatively, the microcrystalline cellulose preferably in the dolutegravir layer in the range of 5.3-6.4 wt/wt% of the uncoated tablet core. The D-Mannitol is preferably present in the dolutegravir layer in an amount of 161.6-159.9 mg per uncoated tablet core. Alternatively, the D-Mannitol preferably in the dolutegravir layer in the range of 18.0-17.8 wt/wt% of the uncoated tablet core.

"Disintegrant" as used herein functions to ensure or facilitate the breakup or disintegration of the composition after administration thereby facilitating dissolution of the active substance. Any suitable disintegrant which is compatible with the active ingredient and to good flow properties and dissolution may be utilized. Exemplary disintegrants include, but are not limited to starch, cellulose and cellulose derivatives such as methyl cellulose, hydroxylpropyl cellulose, hydroxypropylmethyl cellulose, ethyl cellulose, and crosslinked sodium carboxymethyl cellulose, crosslinked polyvinyl pyrrolidone, sodium starch glycolate, agar, bentonite, xanthan gum, and mixtures thereof. Preferably, the disintegrant of the invention is sodium starch glycoate. The disintegrant is preferably present in the range of 35.1-42.9 mg per uncoated tablet core. Alternatively, the disintegrant is preferably in the range of 3.9-4.8 wt/wt% of the uncoated tablet core.

"Lubricant" as used herein is used to prevent adhesion of material to the surface of dies and punches in tablet formation, reduce inter-particle friction, facilitate ejection of tablets from the die cavity, and may improve the flow characteristics of a powder or granules. Any suitable lubricant that is compatible with the active ingredient and to good flow properties and dissolution profile may be utilized. Exemplary lubricants include, but are not limited to talc, magnesium stearate, calcium stearate, zinc stearate, sodium stearyl fumarate, stearic acid, glyceryl behenate, hydrogenated vegetable oils, polyethylene glycol, and mixtures thereof. Preferably magnesium stearate is used as a lubricant in the lamivudine layer of the tablet. Preferably, sodium stearyl fumarate is used as a lubricant extragranular excipient in the tablet formulation. According to one embodiment of the invention, the magnesium stearate is pharmaceutical grade magnesium stearate which is understood to include appreciable amounts of magnesium palmitate and other impurities. According to another embodiment, the magnesium stearate is magnesium stearate per se.

"Binder" as used herein is used to impart cohesiveness qualities to powdered materials so that tablets or granules formed will remain together and not fall apart. Any suitable binder that is compatible with the active ingredient and to good flow properties and dissolution may be utilized. Exemplary binders include, but are not limited to gelatin, starch, cellulose, cellulose derivatives such as methyl cellulose, hydroxylpropyl cellulose, hydroxypropylmethyl cellulose, ethyl cellulose, and carboxymethyl cellulose, sucrose, polyvinyl pyrrolidone (i.e. povidone), natural sugars such as glucose or beta-lactose, corn sweeteners, natural and synthetic gums such as acacia, tragacanth or sodium alginate, polyethylene glycol, waxes and the like. The preferred binder of the invention is povidone. The povidone (ex. povidone K29/32) is preferably used in the dolutegravir layer of the tablet in approximately 1.7 wt/wt% of the uncoated tablet.

As used herein, "AUC" (area under the curve) is the definite integral in a plot of drug concentration in blood plasma vs. time. In practice, the drug concentration is measured at certain discrete points in time and the trapezoidal rule is used to estimate AUC. AUC may be expressed with a time component of 0 to t, wherein t is a certain time, or as 0 to infinity, where the AUC value is extrapolated to infinity based on measurements at certain time intervals.

As used herein, "bilayer" means a unitary dosage form having two distinct layers with each layer having a distinct drug and excipient composition with regard to the other. A bilayer tablet may have an intermediary layer (containing no substantial drug component). Preferably, the two drug-containing layers are in direct contact with one another.

As used herein, "Cmax" is the maximum (or peak) serum concentration that a drug achieves in a specified compartment or test area of the body after the drug has been administrated and before the administration of a second dose.

As used herein, the term "co-administer" refers to administration of two or more agents within a 24-hour period of each other, for example, as part of a clinical treatment regimen. In other embodiments, "co-administer" refers to administration of two or more agents within 2 hours of each other. In other embodiments, "co-administer" refers to administration of two or more agents within 30 minutes of each other. In other embodiments, "co-administer" refers to administration of two or more agents within 15 minutes of each other. In other embodiments, "co-administer" refers to administration at the same time, either as part of a single formulation or as multiple formulations that are administered by the same or different routes.

As used herein, "fasted" describes circumstances under which an individual is dosed and monitored in un unfed condition. The purpose of this is to negate the effect, if any, of fat or other GI content might have on measured pharmacokinetics of drugs being tested. In general, fasting is the state of not having ingested anything other than water for 12 hours prior to initiation of testing, i.e. first dosing. In contrast, "fed" describes circumstances under which an individual has consumed a moderate to high-fat meal prior to first dosing, typically within 4 to 6 hours prior.

"Substantially the same AUC" describes the status of being bioequivalent to a reference product. In this circumstance, regulatory guidelines generally provide a presumption of bioequivalence for a pharmacokinetic parameter in the range of 80%-125% to that of the reference product. For instance, as tested, a test product would be deemed to have substantially the same AUC as a reference product if the measured test AUC falls within 80%-125% of the measured reference AUC.

"Therapeutically effective amount" or "effective amount" refers to that amount of the compound being administered that will prevent a condition, or will relieve to some extent one or more of the symptoms of the disorder being treated. Pharmaceutical compositions suitable for use herein include compositions wherein the active ingredients are contained in an amount sufficient to achieve the intended purpose. Determination of a therapeutically effective amount is well within the capability of those skilled in the art, especially in light of the detailed disclosure provided herein.

As used herein, the term "treatment" or "treating" in the context of therapeutic methods, refers to alleviating the specified condition, eliminating or reducing the symptoms of the condition, slowing or eliminating the progression, invasion, or spread of the condition and reducing or delaying the reoccurrence of the condition in a previously afflicted subject. The present invention further provides use of the compounds of the invention for the preparation of a medicament for the treatment of several conditions in a mammal (e.g., human) in need thereof.

As used herein, the term "prevention" or "preventing" in the context of therapeutic methods, refers to precluding the specified condition or symptoms of the condition, or in the occurrence of prior infection, precluding the re-occurrence of the condition. The present invention further provides use of the compounds of the invention for the preparation of a medicament for the prevention of several conditions in a mammal (e.g., human) in need thereof.

As referenced, the dosage forms herein may be used to treat or, alternatively, prevent HIV which unless further clarified is intended to mean HIV-1. As an alternative embodiment, the combination of the invention may also be effective against HIV-2, or against patients having dual HIV-1/HIV-2 infection.

### EXAMPLES

### Example 1A: Composition of Bi-Layer Tablet

According to an embodiment of the invention there is provided the exemplary bi-layer tablet composition of Table 3.

**Table 3. "Bi-Layer Tablet1"**

| **Component** | **Quantity mg/tablet** | **Function** |
|---|---|---|
| **Layer 1** | | |
| Lamivudine | 300.0 | Active |
| Microcrystalline Cellulose | 277.5 | Filler |
| Sodium Starch Glycolate | 18.0 | Disintegrant |
| Magnesium Stearate | 4.5 | Lubricant |
| Total layer 1 weight per tablet | 600 | - |
| | | |

| **Layer 2** | | |
|---|---|---|
| **Intragranular Excipients (layer 2)** | | |
| Dolutegravir Sodium¹ | 52.6 | Active |
| D-Mannitol² | 145.4 | Diluent |
| Microcrystalline Cellulose | 60.0 | Diluent |
| Povidone | 15.0 | Binder |
| Sodium Starch Glycolate | 15.0 | Disintegrant |
| Purified Water³ | q.s. | Vehicle |
| Total granule amount in layer 2 | 288 | - |

| **Extragranular Excipients (layer 2)** | | |
|---|---|---|
| Sodium Starch Glycolate | 6.0 | Disintegrant |
| Sodium Stearyl Fumarate | 6.0 | Lubricant |
| Total layer 2 weight per tablet | 300 | - |
| Core unit dose (mg) | 900 | - |
| Aquarius Film Coat White (BP18237)⁴ | 18.0 | Film Coat |
| Purified Water³ | q.s. | Vehicle |
| Total unit dose | 918 | - |

| | | |
|---|---|---|
| **Notes:** 1. Equivalent to 50 mg dolutegravir free acid (GSK1349572B) 2. Amount of D-Mannitol may be adjusted to maintain the tablet core weight, based on the purity and water content factor for each batch of drug substance. 3. Purified water is removed during processing. 4. The tablet film coat is applied as a 12% w/w suspension of Aquarius Film Coat in purified water. | | |

### Example 1B: Manufacturing Process for Bi-layer Tablet

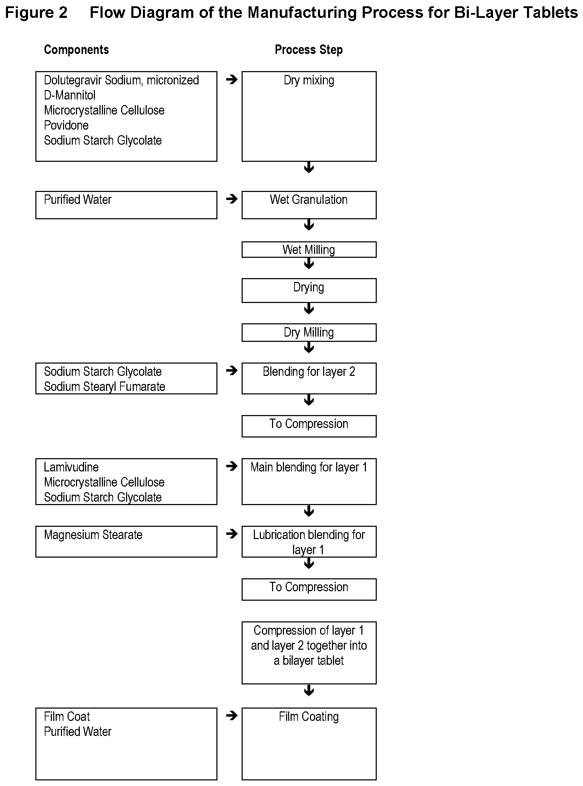

The flow diagram of the manufacturing process of the bi-layer tablets is given in Figure 2.

To create the lamivudine layer compression blend, the lamivudine was blended with microcrystalline cellulose and sodium starch glycolate in a tumble blender. Magnesium stearate was then added to the blend.

To create the dolutegravir layer compression blend, the dolutegravir granule was first made by charging and then mixing the micronised dolutegravir sodium, mannitol, microcrystalline cellulose, povidone K29/32 and sodium starch glycolate in the bowl of a high shear granulator. The mixture was granulated by adding purified water. The wet granules were deagglomerated by passing the wet granules through an impeller mill. Next, the granules were dried in a fluid bed dryer. The dried granules were then milled in an impeller mill.

The dried granules were then blended with sodium starch glycolate and sodium stearyl fumarate in a tumble blender.

Layers 1 and 2 compression blends were compressed using a bilayer rotary tablet press. The layer 1 and 2 filling depths were adjusted to achieve mean layer weights of 600 mg and 300 mg, respectively to give a total bilayer weight of 900 mg. Compression force was adjusted to obtain suitable layer adhesion.

The compressed bilayer tablet cores were then film coated. An aqueous film coating suspension was prepared by dispersing a film coat in purified water in a container. The prewarmed tablet cores were placed in a coating pan, and the coating pan continuously rotated as the tablet cores are sprayed with the coating suspension until the desired weight gain is achieved.

### Reference Example 2: Composition of Mono-Layer Tablet

As described herein is a mono-layer tablet composition for use in comparison with the present invention is provided in Table 4.

**Table 4. "Monolayer Tablet1"**

| **Component** | **Quantity mg/tablet** | **Function** |
|---|---|---|
| **Intragranular Excipients** | | |
| Dolutegravir Sodium¹ | 52.6 | Active |
| D-Mannitol² | 145.4 | Diluent |
| Microcrystalline Cellulose | 60.0 | Diluent |
| Povidone | 15.0 | Binder |
| Sodium Starch Glycolate | 15.0 | Disintegrant |
| Purified Water³ | q.s. | Vehicle |
| Total granule amount per tablet | 288.0 | - |

| **Extragranular Excipients** | | |
|---|---|---|
| Lamivudine | 300.0 | Active |
| Microcrystalline Cellulose | 188.0 | Filler |
| Sodium Starch Glycolate | 24.0 | Disintegrant |
| Sodium Stearyl Fumarate | 20.0 | Lubricant |
| Core unit dose | 820.0 | - |
| Aquarius Film Coat White (BP18237)⁴ | 24.6 | Film Coat |
| Purified Water³ | q.s. | Vehicle |
| Total unit dose | 844.6 | - |

| | | |
|---|---|---|
| **Notes:** 5. Equivalent to 50 mg dolutegravir free acid (GSK1349572B) 6. Amount of D-Mannitol may be adjusted to maintain the tablet core weight, based on the purity and water content factor for each batch of drug substance. 7. Purified water is removed during processing. 8. The tablet film coat is applied as a 12% w/w suspension of Aquarius Film Coat in purified water. | | |

### Example 3: Dissolution Testing

Table 5 summarizes the method and conditions for analysis of tablets as formulated herein.

**Table 5. Dissolution Method Conditions**

| **Dissolution Parameters** | **Value** |
|---|---|
| Apparatus | USP <711> Apparatus II |
| Dissolution medium | Simulated Gastric Fluid (SgF) pH 1.6 |
| Volume | 500 mL |
| Temperature | 37.0 ±0.5°C |
| Sampling Times | 5, 10, 15, 20, 30, 45, 60, 90 minutes |
| Paddle speed | 65 rpm |
| Detection | HPLC |

The proposed dissolution method was used to evaluate the amount and rate of dolutegravir released from various formulations - monolayer tablet of Example 2, the bilayer tablet of Example 1, a commercial grade 50 mg Tivicay^{™} tablet, and a commercial Epivir tablet. The method is not designed to discriminate for lamivudine as it behaves consistently as a very rapidly dissolving tablet.

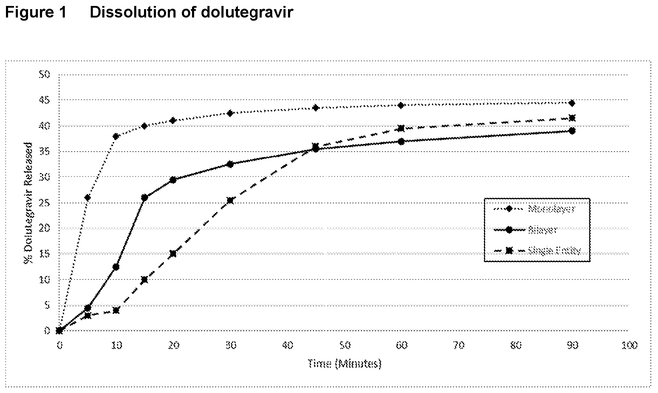

The dissolution test consisted of n =2 for each formulation type. In this experiment the monolayer vs the bilayer formulations were compared against the single entity Tivicay^{™} tablet. To mimic what happens in the clinic where the Tivicay^{™} tablet is dosed with the Epivir^{™} tablet, both single entity components were placed into the dissolution vessel. The dissolution profiles of dolutegravir components according to this method can be seen below in Figure 1. It can be seen that the bi-layer tablet advantageously provides similar 60-minute dolutegravir dissolution as the single entity tablets, i.e. 35-40% dissolution.

### Example 4: Pharmacokinetic testing of Monolayer Tablet 1 and Bilayer Tablet 1

The pharmacokinetics of Monolayer Tablet 1 and Bilayer Tablet 1 were compared to coadministration of the separate agents (the currently U.S. FDA approved single-agent tablets) under fasted conditions, and to assess the effect of a high fat meal on the bioavailability of the FDCs.

This was a 2-part, open-label, single dose, single-center study. Part 1 (N=78) and Part 2 (N=76) were identically designed with 3 periods with a minimum of 7-days washout between periods. Subjects were randomized to receive either the reference or the test FDC (fasted) in a cross-over manner in the first two periods. The first 16 subjects who completed the first two periods and consented to continue, received the test FDC administered with a high fat meal in a third period. Serial pharmacokinetic samples were collected from pre-dose to 72h post-dose. Plasma DTG and 3TC concentrations were assessed using validated LC/MS/MS methods and pharmacokinetic parameters were estimated using noncompartmental methods. The test/reference geometric least squares (GLS) means ratio and associated 90% confidence intervals (CI) of key pharmacokinetic parameters (ln-transformed) were determined using a mixed effects model for DTG and DTG.

In Part 1, 73 subjects completed both periods for the monolayer formulation of Example 2. The GLS means ratios (90%CI) for AUC(0-inf), AUC(0-t) and Cmax were calculated. In Part 2, 74 subjects completed both periods for the bilayer formulation of Example 1. The GLS means ratios (90%CI) for AUC(0-inf), AUC(0-t) and Cmax were calculated. The results are shown in Table 6. All treatments were generally well tolerated.

**Table 6**

| | Example 1 (Bi-layer 1) | Reference Example 2 (Monolayer 1) |
|---|---|---|
| Fasted Conditions | | |
| DTG | 1.1550 (1.0699, 1.2468) | 1.2710 (1.1894, 1.3582) |
| AUC(0-inf) | 1.1578 (1.0718, 1.2507) | 1.2756 (1.1919, 1.3651) |
| AUC(0-t) Cmax | 1.1410 (1.0533, 1.2361), | 1.2805 (1.1890, 1.3790) |

| Fasted Conditions | | |
|---|---|---|
| 3TC | 1.0638 (1.0416, 1.0865) | 1.0341 (1.0097, 1.0591) |
| AUC(0-inf) | 1.0635 (1.0413, 1.0861) | 1.0372 (1.0116, 1.0634) |
| AUC(0-t) | 1.3175 (1.2616, 1.3760) | 1.1956 (1.1437, 1.2498) |
| Cmax | | |

| High Fat Meal DTG | | |
|---|---|---|
| AUC(0-inf) | ~32% increase | ~15% increase |
| AUC(0-t) | ~32% increase | ~15% increase |
| Cmax | No change | No change |

| High Fat Meal 3TC | | |
|---|---|---|
| AUC(0-inf) | No change | No change |
| AUC(0-t) | No change | No change |
| Cmax | ~30% decrease | ~30% decrease |

| | | |
|---|---|---|
| t= tₗₐₛₜ. Median tₗₐₛₜ approximately 72 h for all treatments. | | |

## Claims

1. A bi-layer tablet formulation comprising: (i) a first layer comprising lamivudine, and (ii) a second layer comprising dolutegravir sodium; wherein the formulation contains the quantity of components:
| **Component** | **Range of quantity wt/wt%** |
|---|---|
| Lamivudine | 30.0-36.7 |
| Dolutegravir Sodium | 5.3-6.4 |
| Microcrystalline Cellulose | 33.8-41.3 |
| Sodium Starch Glycolate | 3.9-4.8 |
| Magnesium Stearate | 0.5-0.5 |
| D-Mannitol | 14.5-17.8 |
| Povidone | 1.5-1.8 |
| Sodium Stearyl Fumarate | 0.6-0.7 |
| Purified Water | |

2. The bi-layer tablet formulation according to claim 1, wherein the formulation contains the quantity of components:
| **Component** | **Quantity wt%/wt%** |
|---|---|
| Lamivudine | 33.3 |
| Dolutegravir Sodium | 5.8 |
| Microcrystalline Cellulose | 37.5 |
| Sodium Starch Glycolate | 4.3 |
| Magnesium Stearate | 0.5 |
| D-Mannitol | 16.2 |
| Povidone | 1.7 |
| Sodium Stearyl Fumarate | 0.7 |
| Purified Water | |

3. A bi-layer tablet formulation comprising (i) a first layer comprising lamivudine, and (ii) a second layer comprising dolutegravir sodium; wherein the formulation contains the quantity of components:
| **Component** | **Range of quantity mg/tablet** |
|---|---|
| Lamivudine | 270.0-330.0 |
| Dolutegravir Sodium | 47.3-57.8 |
| Microcrystalline Cellulose | 303.8-371.3 |
| Sodium Starch Glycolate | 35.1-42.9 |
| Magnesium Stearate | 4.1-4.9 |
| D-Mannitol | 130.8-159.9 |
| Povidone | 13.5-16.5 |
| Sodium Stearyl Fumarate | 5.4-6.6 |
| Purified Water | q.s. |

4. The tablet of claim 3, wherein the quantity of components is:
| **Component** | **Quantity mg/tablet** |
|---|---|
| Lamivudine | 300.0 |
| Dolutegravir Sodium | 52.6 |
| Microcrystalline Cellulose | 337.5 |
| Sodium Starch Glycolate | 39.0 |
| Magnesium Stearate | 4.5 |
| D-Mannitol | 145.4 |
| Povidone | 15.0 |
| Sodium Stearyl Fumarate | 6.0 |
| Purified Water | q.s. |

5. The tablet of any one of claims 1-4, further comprising a film coat.

6. A tablet according to any one of claims 1 to 5 for use in medical therapy.

7. A tablet according to any one of claims 1 to 5 for use in treating an HIV infection.

## Patentansprüche

1. Zweischichtige Tablettenformulierung, umfassend: (i) eine erste Schicht, die Lamivudin umfasst, und (ii) eine zweite Schicht, die Dolutegravirnatrium umfasst; wobei die Formulierung die Menge an Komponenten enthält:
| **Komponente** | **Mengenbereich Gew. /Gew.-%** |
|---|---|
| Lamivudin | 30,0-36,7 |
| Dolutegravirnatrium | 5,3-6,4 |
| Mikrokristalline Zellulose | 33,8-41,3 |
| Natriumstärkeglycolat | 3,9-4,8 |
| Magnesiumstearat | 0,5-0,5 |
| D-Mannit | 14,5-17,8 |
| Povidon | 1,5-1,8 |
| Natriumstearylfumarat | 0,6-0,7 |
| Aufgereinigtes Wasser | |

2. Zweischichtige Tablettenformulierung gemäß Anspruch 1, wobei die Formulierung die Menge an Komponenten enthält:
| **Komponente** | **Menge Gew.-%/Gew.-%** |
|---|---|
| Lamivudin | 33,3 |
| Dolutegravirnatrium | 5,8 |
| Mikrokristalline Zellulose | 37,5 |
| Natriumstärkeglycolat | 4,3 |
| Magnesiumstearat | 0,5 |
| D-Mannit | 16,2 |
| Povidon | 1,7 |
| Natriumstearylfumarat | 0,7 |
| Aufgereinigtes Wasser | |

3. Zweischichtige Tablettenformulierung, umfassend (i) eine erste Schicht, die Lamivudin umfasst, und (ii) eine zweite Schicht, die Dolutegravirnatrium umfasst; wobei die Formulierung die Menge an Komponenten enthält:
| **Komponente** | **Mengenbereich mg/Tablette** |
|---|---|
| Lamivudin | 270,0-330,0 |
| Dolutegravirnatrium | 47,3-57,8 |
| Mikrokristalline Zellulose | 303,8-371,3 |
| Natriumstärkeglycolat | 35,1-42,9 |
| Magnesiumstearat | 4,1-4,9 |
| D-Mannit | 130,8-159,9 |
| Povidon | 13,5-16,5 |
| Natriumstearylfumarat | 5,4-6,6 |
| Aufgereinigtes Wasser | q.s. |

4. Tablette gemäß Anspruch 3, wobei die Menge der Komponenten wie folgt ist:
| **Komponente** | **Menge mg/Tablette** |
|---|---|
| Lamivudin | 300,0 |
| Dolutegravirnatrium | 52,6 |
| Mikrokristalline Zellulose | 337,5 |
| Natriumstärkeglycolat | 39,0 |
| Magnesiumstearat | 4,5 |
| D-Mannit | 145,4 |
| Povidon | 15,0 |
| Natriumstearylfumarat | 6,0 |
| Aufgereinigtes Wasser | q.s. |

5. Tablette gemäß irgendeinem der Ansprüche 1-4, die ferner eine Filmbeschichtung umfasst.

6. Tablette gemäß irgendeinem der Ansprüche 1 bis 5 zur Verwendung in der medizinischen Therapie.

7. Tablette gemäß irgendeinem der Ansprüche 1 bis 5 zur Verwendung bei der Behandlung einer HIV-Infektion.

## Revendications

1. Formulation de comprimé bicouche comprenant : (i) une première couche comprenant de la lamivudine, et (ii) une seconde couche comprenant du dolutégravir sodique ; dans laquelle la formulation contient la quantité de constituants :
| **Constituant** | **Plage de quantité % poids/poids** |
|---|---|
| Lamivudine | 30,0 à 36,7 |
| Dolutégravir sodique | 5,3 à 6,4 |
| Cellulose microcristalline | 33,8 à 41,3 |
| Glycolate d'amidon sodique | 3,9 à 4,8 |
| Stéarate de magnésium | 0,5 à 0,5 |
| D-mannitol | 14,5 à 17,8 |
| Povidone | 1,5 à 1,8 |
| Stéarylfumarate de sodium | 0,6 à 0,7 |
| Eau purifiée | |

2. Formulation de comprimé bicouche selon la revendication 1, dans laquelle la formulation contient la quantité de constituants :
| **Constituant** | **Quantité % poids/% poids** |
|---|---|
| Lamivudine | 33,3 |
| Dolutégravir sodique | 5,8 |
| Cellulose microcristalline | 37,5 |
| Glycolate d'amidon sodique | 4,3 |
| Stéarate de magnésium | 0,5 |
| D-mannitol | 16,2 |
| Povidone | 1,7 |
| Stéarylfumarate de sodium | 0,7 |
| Eau purifiée | |

3. Formulation de comprimé bicouche comprenant (i) une première couche comprenant de la lamivudine, et (ii) une seconde couche comprenant du dolutégravir sodique ; dans laquelle la formulation contient la quantité de constituants :
| **Constituant** | **Plage de quantité mg/comprimé** |
|---|---|
| Lamivudine | 270,0 à 330,0 |
| Dolutégravir sodique | 47,3 à 57,8 |
| Cellulose microcristalline | 303,8 à 371,3 |
| Glycolate d'amidon sodique | 35,1 à 42,9 |
| Stéarate de magnésium | 4,1 à 4,9 |
| D-mannitol | 130,8 à 159,9 |
| Povidone | 13,5 à 16,5 |
| Stéarylfumarate de sodium | 5,4 à 6,6 |
| Eau purifiée | q.s. |

4. Comprimé selon la revendication 3, dans lequel la quantité de constituants est :
| **Constituant** | **Quantité mg/comprimé** |
|---|---|
| Lamivudine | 300,0 |
| Dolutégravir sodique | 52,6 |
| Cellulose microcristalline | 337,5 |
| Glycolate d'amidon sodique | 39,0 |
| Stéarate de magnésium | 4,5 |
| D-mannitol | 145,4 |
| Povidone | 15,0 |
| Stéarylfumarate de sodium | 6,0 |
| Eau purifiée | q.s. |

5. Comprimé selon l'une quelconque des revendications 1 à 4, comprenant en outre un pelliculage.

6. Comprimé selon l'une quelconque des revendications 1 à 5, pour une utilisation en thérapie médicale.

7. Comprimé selon l'une quelconque des revendications 1 à 5, pour une utilisation dans le traitement d'une infection par le VIH.
